(19) Europäisches Patentamt
European Patent Office
Office européen des brevets

(11) Veröffentlichungsnummer: **0 250 773**
**A1**

(12) # EUROPÄISCHE PATENTANMELDUNG

(21) Anmeldenummer: 87106377.2

(22) Anmeldetag: 06.09.84

(51) Int.Cl.[3]: C 07 D 319/06

(30) Priorität: 16.09.83 DE 3333413

(43) Veröffentlichungstag der Anmeldung: 07.01.88 Patentblatt 88/1

(84) Benannte Vertragsstaaten: DE FR GB IT

(60) Veröffentlichungsnummer der früheren Anmeldung nach Art. 76 EPÜ: 0 137 309

(71) Anmelder: BAYER AG
Konzernverwaltung RP Patentabteilung
D-5090 Leverkusen 1 Bayerwerk(DE)

(72) Erfinder: Mues, Peter, Dr.
Weihers Hecke 30
D-4100 Duisburg 46(DE)

(72) Erfinder: Brassat, Bert, Dr.
Bodelschwinghstrasse 30
D-4150 Krefeld(DE)

(72) Erfinder: Buysch, Hans-Josef, Dr.
Brandenburger Strasse 28
D-4150 Krefeld(DE)

(54) **Neue Chlorkohlensäureester.**

(57) Neue Chlorkohlensäureester der Formel

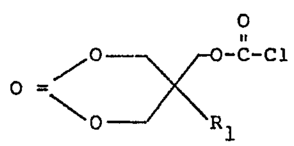

(I)

EP 0 250 773 A1

Teilanmeldung aus der
europäischen Patentanmeldung 84 110 591.9



BAYER AKTIENGESELLSCHAFT 5090 Leverkusen, Bayerwerk

Konzernverwaltung RP
Patentabteilung

Neue Chlorkohlensäureester

Die vorliegende Erfindung betrifft neue Chlorkohlensäureester der Formel (I)

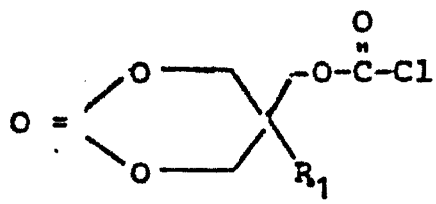

(I),

in der

$R_1$ für einen Alkylrest mit 1 bis 4 C-Atomen steht. Vorzugsweise steht $R_1$ für eine Methyl- oder Ethylgruppe.

Die Herstellung der neuen Chlorkohlensäureester der Formel (I) kann durch Phosgenierung der den neuen Chlorkohlensäureestern zugrunde liegenden Alkohole der Formel (II)

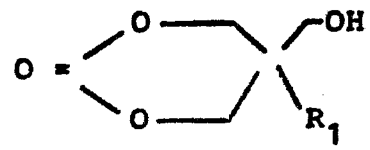 (II),

in der

$R_1$ die oben angegebene Bedeutung hat,

nach einem an sich bekannten Verfahren erfolgen. Die Alkohole der Formel (II) können gemäß dem in der DE-OS 3 103 139 beschriebenen Verfahren hergestellt werden.

Die Phosgenierung der Alkohole der Formel (II) kann beispielsweise so erfolgen, daß man Phosgen in einem Lösungsmittel vorlegt und den Alkohol der Formel (II) zusammen mit einem säurebindenden Mittel nach und nach bei Temperaturen im Bereich von -5 bis +5°C zufügt. Phosgen wird dabei vorzugsweise im Überschuß eingesetzt, beispielsweise in 5 bis 20%-igem molarem Überschuß. Lösungsmittel können beispielsweise chlorierte Kohlenwasserstoffe sein, wie Methylenchlorid. Als säurebindende Mittel kommen beispielsweise aromatische oder aliphatische Amine in Frage, wie N,N-Dimethylanilin, Pyridin oder Triethylamin. Beispiele für einzusetzende Alkohole der Formel (II) sind Trimethylolethancarbonat, Trimethylolpropancarbonat, Trimethylolbutancarbonat und Trimethylolpentancarbonat.

Die Isolierung der neuen Chlorkohlensäureester der Formel (I) aus dem Phosgenierungs-Reaktionsgemisch kann beispielsweise dadurch erfolgen, daß man aus dem Reaktionsgemisch alle leicht flüchtigen Anteile durch Vakuumdestillation entfernt, den Rückstand in einem Lösungsmittel für die Chlorkohlensäureester der Formel (I), z.B. Toluol, aufnimmt, unlösliche Bestandteile abtrennt und das Lösungsmittel wieder entfernt.

Aus den neuen Chlorkohlensäureestern der Formel (I) lassen sich neue mehrcyclische Kohlensäureester der Formel (III)

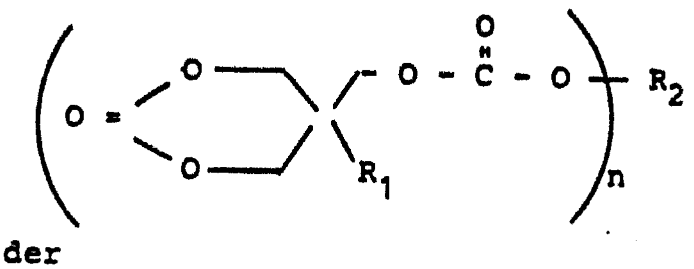
(III),

in der

$R_1$ für einen Alkylrest mit 1 bis 4 C-Atomen steht,

$R_2$ für einen Alkylidenrest mit 3 bis 18 C-Atomen, für einen Arylrest mit 6 bis 12 C-Atomen oder für einen Aralkylrest mit 7 bis 24 C-Atomen steht und

n 2, 3 oder 4 bedeutet,

herstellen. Die neuen mehrcyclischen Kohlensäureester der Formel (III) sind Gegenstand der Stammanmeldung.

In der Formel (III) steht $R_1$ vorzugsweise für Methyl oder Ethyl und $R_2$ vorzugsweise für das Kohlenwasserstoffgerüst eines der folgenden 2- bis 4-wertigen Alkohole: Trimethylolethan, Trimethylolpropan, Pentaerythrit, Butandiol-1,4, Buten-2-diol-1,4, Butin-2-diol-1,4, Neopentylglykol, Hexandiol-1,6, 2-Methylen-propandiol-1,3,

Cyclohexandiol-1,4, Cyclohexan-1,4-dimethanol, Bisphenol A und Perhydrobisphenol A. n entspricht dann der Wertigkeit des Alkohols, dessen Kohlenwasserstoffgerüst den Rest $R_2$ darstellt. Besonders bevorzugt ist $R_2$ das Kohlenwasserstoffgerüst eines 2- oder 3-wertigen Alkohols, ganz besonders bevorzugt das Kohlenwasserstoffgerüst eines zweiwertigen Alkohols, d.h. n steht besonders bevorzugt für 2 oder 3 und ganz besonders bevorzugt für 2.

Die neuen mehrcyclischen Kohlensäureester der Formel (III) können hergestellt werden, indem man Chlorkohlensäureester der Formel (I) zu einer Lösung, die einen 2- bis 4-wertigen Alkohol und ein säurebindendes Mittel enthält, nach und nach bei Temperaturen im Bereich von 0 bis 30°C zufügt.

Zur Herstellung der neuen mehrcyclischen Kohlensäureester der Formel (III) ist es nicht unbedingt erforderlich, die Chlorkohlensäureester der Formel (I) in reiner Form einzusetzen; sie können beispielsweise auch in Form der bei ihrer Herstellung anfallenden Roh-Lösung eingesetzt werden. Die 2- bis 4-wertigen Alkohole sind solche, deren Kohlenwasserstoffgerüst einen Alkylidenrest mit 3 bis 18 C-Atomen, einen Arylrest mit 6 bis 12 C-Atomen oder einen Aralkylrest mit 7 bis 24 C-Atomen darstellt. Vorzugsweise handelt es sich bei den 2- bis 4-wertigen Alkoholen um einen Alkohol aus der Gruppe Trimethylolethan, Trimethylolpropan, Pentaerythrit, Butandiol-1,4, Buten-2-diol-1,4, Butin-2-diol-1,4, Neopentylglykol, Hexandiol-1,6, 2-Methylen-propandiol-1,3, Cyclohexandiol-1,4, Cyclohexan-1,4-dimethanol, Bisphenol A und Perhydrobisphenol A. Als säurebindende Mittel kommen beispielsweise aromatische oder aliphatische Amine in Frage, wie N,N-Dimethylanilin, Pyridin oder Triethylamin. Lösungsmittel können beispielsweise chlorierte Kohlenwasserstoffe sein, wie Methylenchlorid.

Die Isolierung und Reinigung der neuen mehrcyclischen Kohlensäureester der Formel (III) kann beispielsweise erfolgen, indem man aus dem Reaktionsgemisch zunächst das aus dem säurebindenden Mittel entstandene Hydrochlorid mit Wasser oder einer wäßrigen Salzlösung extrahiert, aus der verbleibenden organischen Phase, gegebenenfalls nach einer Trocknung, das Lösungsmittel abtrennt und das so erhaltene Rohprodukt aus einem geeigneten Lösungsmittel, z.B. Butylacetat, kristallisiert.

Aus den mehrcyclischen Kohlensäureestern der Formel (III) können durch Copolymerisation mit monocyclischen Carbonaten neue Copolymere erhalten werden. Solche neuen Copolymere sind ebenfalls Gegenstand der Stammanmeldung. Als monocyclische Carbonate können in die Copolymerisation beispielsweise solche eingesetzt werden, die den Formeln (IV), (V) oder (VI) entsprechen.

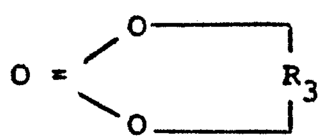

(IV) .

$R_3$ steht für $-(CH_2)_m-$, wobei m für 3, 4, 5 oder 6 steht, $-CH_2-CH{=}CH-CH_2-$, $-CH_2-CH_2-CH(CH_3)-$, $-CH_2-CH_2-O-CH_2-CH_2-O-CH_2-CH_2-$,

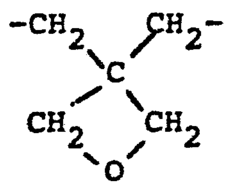 , 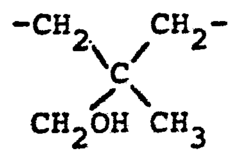 , 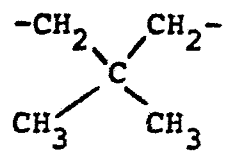 oder

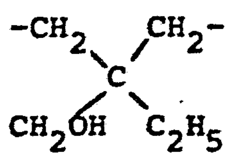 .

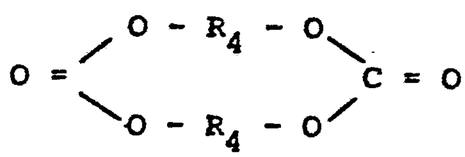 (V)

$R_4$ steht für $-(CH_2)_p-$, wobei p eine ganze Zahl von 4 bis 12 bedeutet,
$-CH_2-CH_2-O-CH_2-CH_2-$, $-CH_2-CH_2-O-CH_2-CH_2-O-CH_2-CH_2-$ oder $-CH_2-CH_2-N(CH_3)-CH_2-CH_2-$.

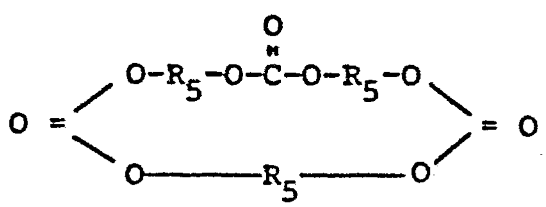 (VI)

$R_5$ steht für $-CH_2-CH_2-O-CH_2-CH_2-$.

Die Herstellung derartiger monocyclischer Carbonate ist beispielsweise in den DE-OS'en 31 03 135, 31 03 139, 31 03 140 und 32 04 078 beschrieben.

Vorzugsweise werden monocyclische Carbonate der Formel (IV) eingesetzt, besonders bevorzugt 5,5-Dimethyl-1,3-dioxan-2-on.

Bei der Copolymerisation der neuen mehrcyclischen Kohlensäureester der Formel (III) mit den monocyclischen Carbonaten wird der Ring der monocyclischen Carbonate geöffnet, und es entstehen lineare Polycarbonate, die durch von den neuen mehrcyclischen Kohlensäureestern der Formel (III) stammenden Struktureinheiten vernetzt sind. Diese Copolymere sind Duromere mit verbesserten Eigenschaften, insbesondere mit überraschend hohen Schlagzähigkeiten.

Zur Copolymerisation werden beispielsweise 2 bis 50, vorzugsweise 5 bis 20 Gew.-% eines mehrcyclischen Kohlensäureesters der Formel (III) und 98 bis 50, vorzugsweise 95 bis 80 Gew.-% eines monocyclischen Carbonats eingesetzt (diese Gewichtsprozente beziehen sich jeweils auf die Gesamtmenge eingesetzter organischer Carbonate). Die Copolymerisation wird im allgemeinen bei erhöhter Temperatur durchgeführt, beispielsweise bei Temperaturen im Bereich von 80 bis 200°C. Bevorzugt sind Temperaturen im Bereich von 100 bis 160°C. Besonders bevorzugt sind Temperaturen im Bereich von 110 bis 140°C. Die Copolymerisation wird ferner im allgemeinen unter Zusatz von Katalysatoren durchgeführt. Geeignete Katalysatoren sind beispielsweise die Carbonate und Carboxylate der Metalle Lithium, Natrium, Kalium, Zinn, Blei und Thallium. Bevorzugt sind Carboxylate von Lithium, Blei und Thallium. Die Katalysatoren können beispielsweise in Mengen von 0,01 bis 0,1 Gew.-%, bezogen auf die Gesamtmenge eingesetzter Carbonate, eingesetzt werden. Bevorzugt sind Katalysatormengen von 0,01 bis 0,05 Gew.-%.

Die durch diese Copolymerisation zugänglichen Duromere können mit Vorteil überall dort eingesetzt werden, wo hohe Anforderungen an duromertypische Eigenschaften, wie Wärmeformbeständigkeit und Unempfindlichkeit gegen Agentien, die eine Lösung oder Quellung hervorrufen können (z.B. Kraft- und Schmierstoffe), gestellt werden und, wo zusätzlich gute mechanische Eigenschaften, insbesondere eine hohe Zähigkeit notwendig sind. Beispielsweise können Kraftfahrzeugteile aus derartigen Duromeren hergestellt werden, insbesondere solche, die der Gefahr von Steinschlag ausgesetzt sind, wie Spoiler, Stoßstangen(teile) und Kotflügel(auskleidungen).

Die folgenden Beispiele erläutern die Erfindung, ohne sie in irgendeiner Weise zu beschränken.

## Beispiele

### Beispiel 1

Zu einer Lösung von 53 g (0,535 Mol) Phosgen in 200 ml getrocknetem Methylenchlorid wurde bei 5°C innerhalb von 40 Min. eine Lösung von 80 g (0,5 Mol) 1,3-Dioxan-5-hydroxymethyl-5-ethyl-2-on und 39,5 g (0,5 Mol) Pyridin in 200 ml getrocknetem Methylenchlorid zugetropft. Die Mischung wurde bei 0°C während weiterer 3 Stunden gerührt. Anschließend wurden im Wasserstrahlvakuum 100 ml Methylenchlorid abdestilliert. Das zum Teil ausfallende Pyridinhydrochlorid wurde abgesaugt und mit wenig Methylenchlorid gewaschen. Das Filtrat wurde mittels eines Rotationsverdampfers bei 0°C eingeengt. Der kristalline Rückstand (163 g) wurde 3-mal mit insgesamt 1,6 l Toluol verrührt und die ungelösten Bestandteile abgesaugt. Das Filtrat wurde mittels eines Rotationsverdampfers bei 30°C eingeengt. Es verblieben 79 g (71 % der Theorie) kristallines Produkt mit einem Schmelzpunkt von 62 bis 65°C.

Das $^1$H-NMR-Spektrum und die analytische Chlorbestimmung bestätigten die Struktur und Zusammensetzung des erhaltenen Produkts als eine Verbindung der Formel (I) mit $R_1 = C_2H_5$.

### Beispiel 2

Zu einer Lösung von 110 g (1,1 Mol) Phosgen in 400 ml getrocknetem Methylenchlorid wurden 160 g (1 Mol) 1,3-Dioxan-5-hydroxymethyl-5-ethyl-2-on gelöst in 95 g

(1,2 Mol) Pyridin und 700 ml getrocknetem Methylenchlorid innerhalb von 2 Stunden bei 4°C zugetropft. Nach weiteren 2 Stunden wurde die erhaltene Lösung zu einer Mischung aus 82 g (0,75 Mol) Cyclonexan-1,4-dimethanol, 79 g (1 Mol) Pyridin und 200 ml Methylenchlorid bei 20°C innerhalb von 25 Min. gegeben. Nach 15 Stunden bei Raumtemperatur wurde 3-mal mit insgesamt 1 l gesättigter wäßriger NaCl-Lösung extrahiert. Die organische Phase wurde über $Na_2SO_4$ getrocknet und mittels eines Rotationsverdampfers schonend eingeengt (Badtemperatur 40°C). Restliches Lösungsmittel wurde im Vakuum einer Ölpumpe entfernt. Das Rohprodukt (286 g) wurde in 900 ml heißem Butylacetat gelöst. Beim Abkühlen auf Raumtemperatur und später auf 5°C fielen 155 g Produkt (60 % der Theorie bezogen auf eingesetztes 1,3-Dioxan-5-hydroxymethyl-5-ethyl-2-on mit einem Schmelzpunkt von 95 - 102°C) aus.

Das $^1$H-NMR-Spektrum bestätigte die Struktur und Zusammensetzung des erhaltenen Produkts als einer Verbindung der Formel (III) mit $R_1 = C_2H_5$, $R_2 = -CH_2-$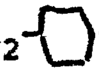$-CH_2-$ und $n = 2$.

<u>Beispiel 3</u>

Es wurde verfahren wie in Beispiel 2, jedoch wurde das Cyclohexan-1,4-dimethanol durch die entsprechende Menge 2,2-Dimethylpropandiol-1,3 (= Neopentylglykol) ersetzt. Es wurde ein Produkt mit einem Schmelzpunkt von 74°C erhalten. Das $^1$H-NMR-Spektrum bestätigte die Struktur des erhaltenen Produkts als einer Verbindung der Formel (III) mit $R_1 = C_2H_5$,

$R_2 = -CH_2-\overset{CH_3}{\underset{CH_3}{C}}-CH_2-$ und n = 2.

Beispiel 4

Es wurde verfahren wie in Beispiel 2, jedoch wurde das Cyclohexan-1,4-dimethanol durch die entsprechende Menge Perhydrobisphenol A ersetzt. Es wurde ein Produkt mit einem Schmelzpunkt von 190 bis 200°C erhalten. Das $^1$H-NMR-Spektrum bestätigte die Struktur des erhaltenen Produkts als einer Verbindung der Formel (III) mit $R_1 = C_2H_5$,

$R_2$ = 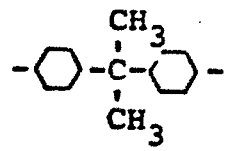 und n = 2.

Beispiel 5

Es wurde verfahren wie in Beispiel 2, jedoch wurde das Cyclohexan-1,4-dimethanol durch die entsprechende Menge Bisphenol A ersetzt. Es wurde ein Produkt mit einem Schmelzpunkt von 132 - 135°C erhalten. Das $^1$H-NMR-Spektrum bestätigte die Struktur des erhaltenen Produkts als einer Verbindung der Formel (III) mit $R_1 = C_2H_5$,

$R_2$ = 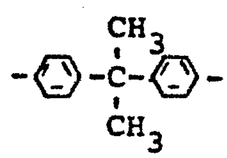 und n = 2.

Beispiel 6

153 g (1,18 Mol) 5,5-Dimethyl-1,3-dioxan-2-on (Formel

(IV), $R_3$ = 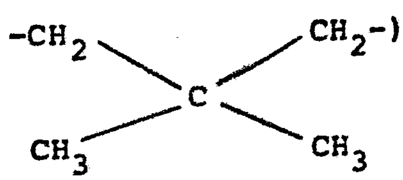 und 17 g (0,0033 Mol)

5,5'-(Cyclohexyl-1,4-bis-(methylenoxy-carboxy(oxymethylen)) bis(5-ethyl-1,3-dioxan-2-on) (Formel (III), $R_1 = C_2H_5$, $R_2 = -CH_2-$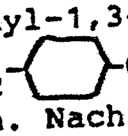$-CH_2-$ und n = 2) wurden bei 120°C aufgeschmolzen. Nach Zugabe von 17 mg Thalliumacetat wurde auf 125°C erhitzt. Nach 20 Minuten trat eine Polymerisation zu einem unschmelzbaren, in Lösungsmitteln unlöslichen, transparenten Polymerisat von großer Zähe, Härte und Elastizität ein. Das Polymerisat wurde weitere 30 Minuten bei 125°C getempert.

Beispiel 7

Die Eigenschaften des Produktes aus Beispiel 6 und im Vergleich dazu eines Produktes, das nach dem Stand der Technik (DE-OS 31 03 139) aus 90 Gew.-% 5,5-Dimethyl-1,3-dioxan-2-on und 10 Gew.-% des Carbonates mit der Formel

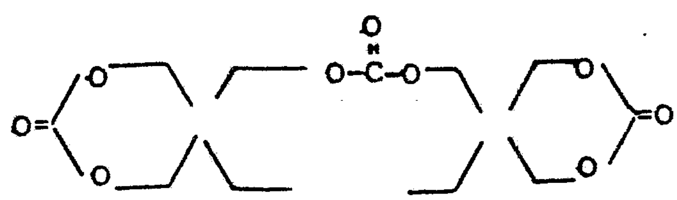

hergestellt wurde, wurden bestimmt. Die Ergebnisse sind aus Tabelle 1 ersichtlich.

Tabelle 1

| | | Stand der Technik | Produkt aus Beispiel 6 |
|---|---|---|---|
| Zugfestigkeit | R (MPa) | 26 | 30 |
| Reißdehnung | R (%) | 12,7 | 13,6 |
| Streckspannung | S (MPa) | 22,2 | 30 |
| Streckdehnung | S (%) | 2,5 | 3,1 |
| 3,5 %-Biegespannung | b3,5 (MPa) | 42,8 | 59 |
| Schlagzähigkeit | $a_n$ (kJ/$m^2$) | 24,3 | 46 |
| E-Modul (Zug) | (MPa) | 1630 | 1850 |

0250773

Patentansprüche:

1. Neue Chlorkohlensäureester der Formel

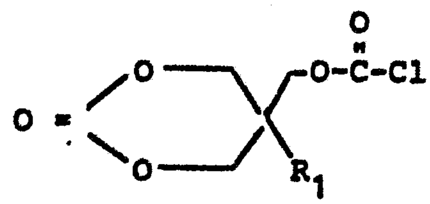

in der
$R_1$ für einen Alkylrest mit 1 bis 4 C-Atomen steht.

2. Neue Chlorkohlensäureester der im Anspruch 1 angegebenen Formel, wobei $R_1$ für Methyl oder Ethyl steht.

Europäisches Patentamt

# EUROPÄISCHER RECHERCHENBERICHT

Nummer der Anmeldung
EP 87 10 6377

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
|---|---|---|---|
| D,A | EP-A-0 057 360 (BAYER)<br>* Seite 27; Ansprüche *<br>----- | 1,2 | C 07 D 319/06 |
| | | | RECHERCHIERTE SACHGEBIETE (Int Cl 4) |
| | | | C 07 D 319/00 |

Der vorliegende Recherchenbericht wurde für alle Patentansprüche erstellt.

| Recherchenort | Abschlußdatum der Recherche | Prüfer |
|---|---|---|
| DEN HAAG | 09-01-1985 | FRANCOIS J.C.L. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82